# EUROPEAN PATENT APPLICATION

(11) **EP 3 326 529 A1**
(43) Date of publication of application: **30.05.2018**
(21) Application number: 16757724.6
(22) Date of filing: 21.07.2016
(51) Int. Cl.: A61B 5/145, A61B 5/05, A61B 5/00

(54) **TRANSDUCER FOR MEASURING GLUCOSE IN BLOOD IN A NON-INVASIVE MANNER**

(30) Priority: 21.07.2015 AR P150102315
(71) Applicant: Inis Biotech LLC, Kent County, DE 19963 (US); Consejo Nacional de Investigaciones Científicas Y Técnicas (CONICET), Buenos Aires C1425FQB (AR)
(72) Inventor: CASTIÑEIRA MOREIRA, Jorge, Mar del Plata - Pcia. de Buenos Aires B7605AGD (AR); AGÜERO, Pablo Daniel, Mar del Plata - Pcia. de Buenos Aires B7608HKM (AR); BONADERO, Juan Carlos, Mar del Plata - Pcia. de Buenos Aires B7602CIF (AR); URIZ, Alejandro José, Mar del Plata - Pcia. de Buenos Aires B7611BDH (AR); RABIOGLIO, Lucas Andrés, Necochea - Pcia. de Buenos Aires B7632FMB (AR); CEBEDIO, María Celeste, General Madariaga - Pcia. de Buenos Aires B7163EBJ (AR)
(74) Representative: Zardi, Marco
(86) International application number: PCT/IB2016/054351
(87) International publication number: WO 2017/013616

(57) **Abstract**

A system for noninvasive measurement of glucose in blood. The system is based in a sensor of the invention. The user must lay its finger on said sensor, which is excited by means of a radiofrequency sweep generation. The responses of magnitude and phase as a function of the frequency of the reflected wave are measured by an amplitude and phase detector. Said magnitudes are acquired by two analog-to-digital converters of a microcontroller, which in turn has the ability of transmitting them to other devices such as for example a computer or a smart phone.

## Description

### FIELD OF THE INVENTION

The present invention refers to a transducer that allows for the noninvasive measurement of glucose in blood and particularly refers to an integrated equipment for the measurement of glucose in blood in a noninvasive manner.

### BACKGROUND OF THE INVENTION

Diabetes is a chronic condition that arises when the organism loses its ability of producing enough amounts of insulin or of using it effectively in order to maintain glucose levels in the blood within normal margins. Insulin is a hormone produced by the pancreas which allows the glucose from food to enter the organism's cells, where it is converted into energy for keeping muscles and tissues functioning. As a result, a person with diabetes does not absorb glucose properly, so it remains circulating in the blood (hyperglycemia) and damaging the tissues over time, which may cause damage to the eyes, the kidneys, the nerves, heart diseases, strokes. These complications can be reduced between 40% and 75% by appropriate control of the glucose levels in blood.

Diabetes is a common chronic and costly disease that appears when the pancreas does not produce enough insulin or when the organism does not use the produced insulin in an efficient way. It is one of the four priority noncommunicable diseases identified by the World Health Organization (WHO), along with cardiovascular disease (which includes myocardial infarction and stroke), cancer and chronic respiratory disease. It is related to rapid increase in overweight, obesity and physical inactivity; it has a genetic component and some people simply are more susceptible than others to develop diabetes. Diabetes is at crisis levels and continues to rise. Every seven seconds, a person dies from diabetes, which means that four million deaths occur worldwide each year. In 2012, 347 million people suffered from diabetes and there are 280 million at high risk of developing it. If nothing is done, the number of people with diabetes will rise up to 522 million in 20 years, with another 398 million people in high risk, and in 2030 it could become the seventh cause of death worldwide. Likewise, 80% of the deaths by diabetes are registered in countries with low and medium income. In Latin America there are about 15 million people with diabetes mellitus and this number will reach 20 million in 10 years, many more than is to be expected from simple population growth. This epidemic behavior is probably due to various factors, among which race, life habit changes and aging stand out. In Argentina, it is estimated that there are two and a half million people with diabetes. According to projections, it would reach 4 million Argentines in the year 2020.

Currently there are numerous devices that allow for controlling the levels of glucose in blood, but in general these require a puncturing somewhere in the body in order to extract a blood sample. The invasive nature of conventional methods, is painful and often causes most patients to no perform an adequate control. While there are some companies which have dabbled in noninvasive glucose measuring, there is still no technique that is widely adopted and its application is at the moment in experimental state.

### BRIEF DESCRIPTION OF THE INVENTION

It is thus an object of the present invention to provide a device for the noninvasive measurement of glucose in blood which can be utilized by the person suffering from diabetes, both type I and type II, for monitoring the levels of glucose in blood, and perform a more adequate control of the disease, allowing for a more adequate diet, and in the case of diabetes type I, determining in a much better way the application of the necessary insulin doses. The use of a measuring device of this type also avoids the repeated use of invasive measurements which typically involve puncturing, and the need for reactive strips which in general are costly.

It is therefore an object of the present invention to provide a system for noninvasive measurement of the glucose levels in blood. The system is based on a sensor of the invention representing the finger, which has been designed based on a novel layered model of the human finger. The user must lay his finger over the designed sensor, which is excited by means of a radio frequency sweep generator. The responses of magnitude and phase as a function of the frequency of the reflected wave are measured by an amplitude and phase detector. In the proposed implementation, said magnitudes are acquired by two of the analog-to-digital converters of a microcontroller, which in turn has the ability to transmit them to other devices such as for example; a computer or a smart phone.

Accordingly, it is an object of the present invention a device for the measurement of glucose in blood in a noninvasive manner, which comprises a reflected wave meter and a sensor, the reflected wave meter comprising a sweep generator based on integrated circuits for synthetizing a wave that hits and is reflected in the sensor; and a dual directional coupler for receiving the reflected wave.

In a preferred embodiment of the present invention, the dual directional coupler generates an output signal that is sent to a reflected wave amplitude and phase detector which generates corresponding amplitude and phase output signals.

In a preferred embodiment of the present invention, said sensor is a resonator.

In a preferred embodiment of the present invention, the operation of the system is controlled by means of a microcontroller, which controls the sweep of the sweep generator and receives said amplitude and phase output signals from the amplitude and phase detector.

In a preferred embodiment of the present invention, said microcontroller is in turn connected to an LCD display and/or to a wireless transmission module.

### DESCRIPTION OF THE DRAWINGS

For better clarity and understanding of the object of the present invention, the same has been illustrated in several figures, wherein it has been depicted in one of the preferred embodiments, as a way of example, wherein:

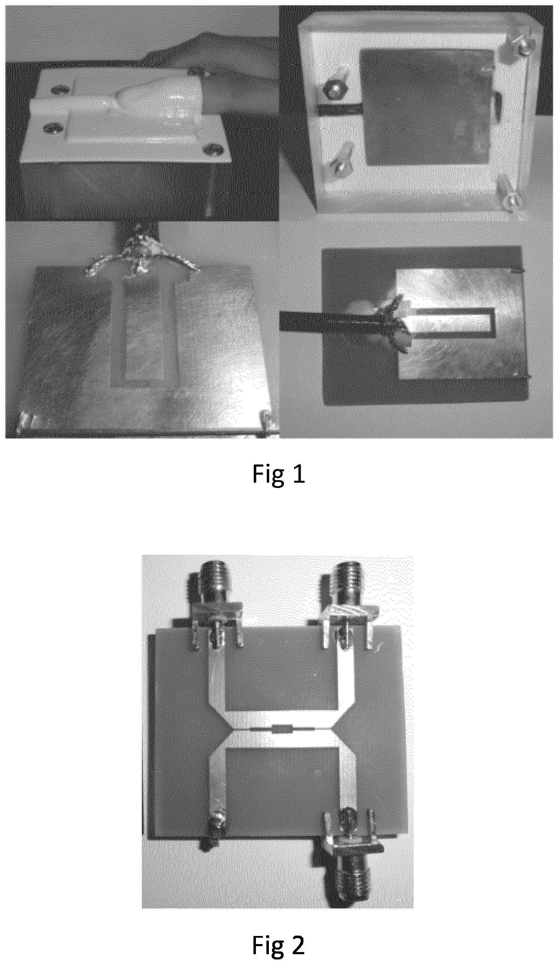

Figure 1 shows an implementation of the resonator in the device of the present invention;
Figure 2 is an image of the dual directional coupler used to measure the reflected electromagnetic wave of the device of Figure 1;

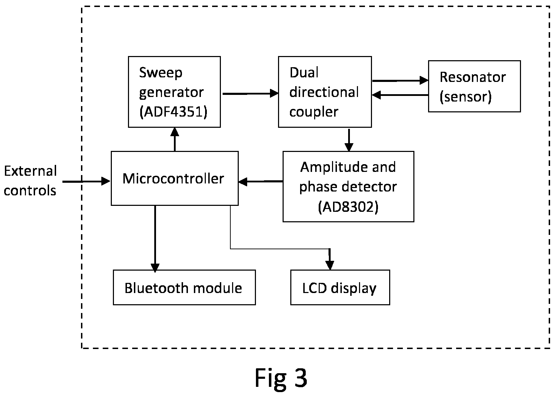

Figure 3 is a block diagram of the full implementation of the measuring system. The block labeled "resonator (sensor)" is the device shown in Figure 1, while the dual directional coupling is shown in Figure 2.

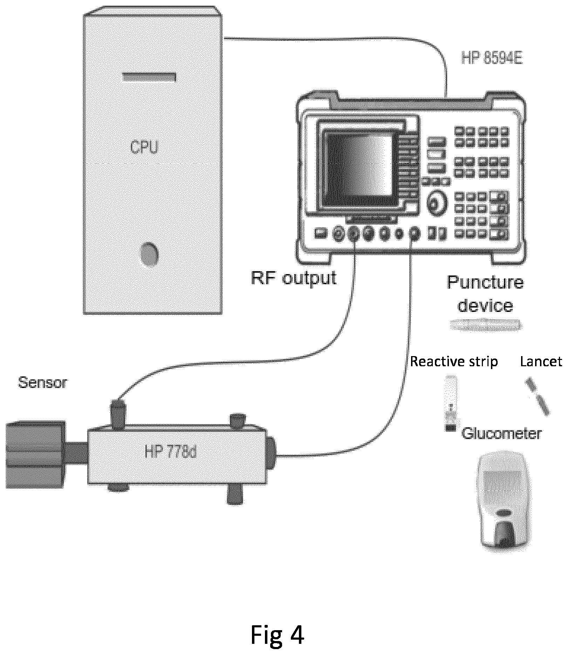

Figure 4 illustrates the test bench used for analyzing the performance of the resonator of the invention.
Figure 5 shows the human finger model used to design and simulate the resonator presented in Figure 1.

### DETAILED DESCRIPTION OF THE EMBODIMENT

The device consists of a reflected wave meter and a sensor of the invention.

For this, a sweep generator is required to synthesize the wave that will hit the sensor. This sweep generator for the working prototype will be based on the integrated circuit ADF4351 from Analog Devices. Then, a dual directional coupler will be used (also developed exclusively for this application).

The complete measuring device also involves the preliminary design of a sweeper of frequencies up to 2.8 GHz, a reflectometer for measuring the reflection parameter of the input of the resonator-finger system, based on the design of a dual directional coupler. The implementation of an ultrasound thickness measurer which has been used to determine with greater accuracy the thickness of the layers of human tissue which form part of the model of the human finger used has also been postulated. The output signal of the dual directional coupler is sent to a reflected wave amplitude and phase detector.

Although the level of glucose in blood is currently estimated based on the measurements of the amplitude of the reflected wave, there are studies which indicate that there might be information of interest in the phase of the reflected wave, for that reason, foreseeing the continuation of the investigation, the integrated circuit AD8302 from Analog Devices was used, which allows detecting these two magnitudes within the range of frequencies of interest.

The operation of the system will be controlled by means of a microcontroller (in the conducted implementation, one from the firm Microchip was used), which will be responsible for controlling the sweep of the ADF4351 and receive the amplitude and phase signals coming from the AD8302. Additionally, it will be used as a user interface, as it will comprise external controls, a liquid crystal screen or LCD display for presenting the obtained results, and a Bluetooth link or a wireless transmission module in one of its serial ports, which will allow it to communicate with a computer or smartphone.

The measurement bench used comprises a Spectrum Analyzer HP8594 E which also comprises sweep generator of frequencies reaching up to 2.9 GHz. This signal is used as the incident wave of the resonator-finger system, which acts as a load connected through a dual directional coupler HP 778d, from which the reflected wave originates, which is connected to the input of the Spectrum analyzer to observe its variation in relation to the frequency, and thus determine its resonance frequency. In the measurement procedure of the experiment, the individual ingests some type of food of high glycemic index to elevate the levels of sugar in blood. The individual of the case of the experiment is a healthy individual, who does not suffer from diabetes, and for whom the plastic fixing support for the posture of the fifth digit (little finger or pinky finger) was custom built. In 5 minute intervals, the measurements of the frequency response where carried out and at the same time the glucose values where determined by means of a conventional invasive measurer. Taking into account that conventional invasive measurers determine the levels of glucose in blood with an error of +- 10 %, the measurements resulted in a linear correlation between the resonance frequency values and glucose, for the individual subjected to the measurement.

The designed method is based on electromagnetic simulation by means of computer programs, and in this simulation of the resonator-finger system, the electrical model of the human finger, which is represented by a layered model, that simulate the electrical characteristics of different tissues, epidermis, dermis, muscle, fat, blood and bone is critical. In order to carry out this model, initially some classic parameters obtained from literature where used, however it was then discovered that the current model of the finger had to be improved, given by the discrepancies found between the computer simulation and the experimental measurements. In this process of improving the model the dimensions of its layers where determined from ultrasound images of the human finger, with the help of a professional. In order to establish the dielectric properties of each layer, a Debye model, widely used for characterizing biological tissues or any other sample presenting a dispersive nature among its intrinsic electrical properties, was used.

Once the living tissue has been modeled, a thorough study of the planar structures used in microwaves is performed, in order to evaluate which structure will result most appropriate. In this case, three different technologies where studied: microstrip structures, coplanar structures and coplanar structures with ground plane.

Due to the inhomogeneous characteristic of the sample and the existing variability between individuals, the analysis took into account not only the sensitivity to the changes in glycaemia, but also the invariance of parameters such as skin thickness, finger size, etc. As a result of the analysis is was concluded that the technology to be implemented is a coplanar sensor with a ground plane. The same was implemented in practice, and in the real implementation a support built in plastic was added to guarantee certain degree of repeatability in the measurements carried out regarding the form of positioning the finger over the transducer.

The measurements where satisfactory, validating the performed simulations and asserting the correlation between the sensor response and the glycaemia level of the sample, which is the principle that gave origin to this invention. A high dependency of the associated factors was also observed, which leads to a dependency of the measurement in relation to the characteristics of the individual, which appears as a problem to be solved.

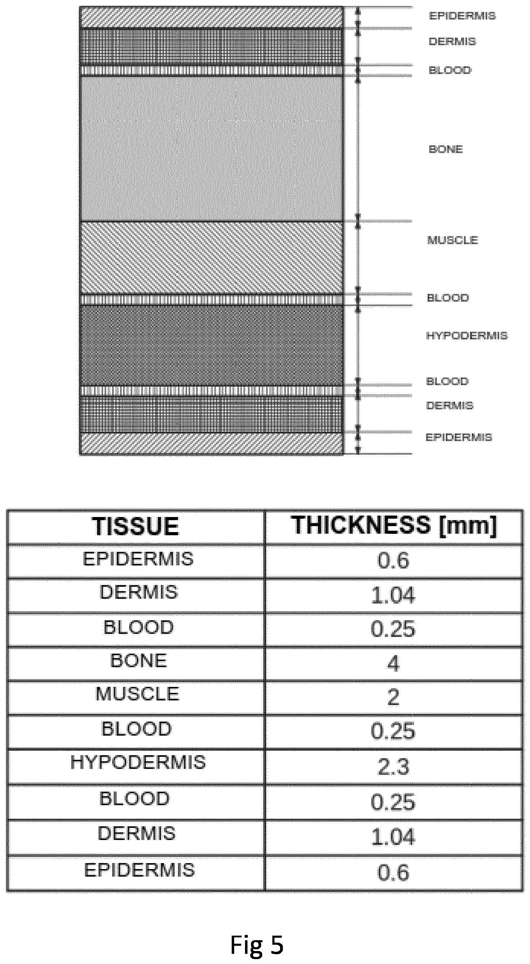

In reference to Figure 5, each of the materials that make up the finger model and their corresponding thicknesses are presented. These thicknesses are not used in the literature, but where obtained by means of the respective clinical studies.

## Claims

1. A device for noninvasive measurement of glucose in blood, comprising a reflected wave meter and a sensor, the reflected wave meter comprising a sweep generator based on integrated circuits in order to synthesize a wave that hits and is reflected in the sensor; and a dual directional coupler for receiving the reflected wave.

2. The device according to claim 1, wherein the dual directional coupler generates an output signal that is sent to a reflected wave amplitude and phase detector which generates corresponding amplitude and phase output signals.

3. The device according to claim 1, wherein said sensor is a resonator.

4. The device according to claim 2, wherein operation of the system is controlled by means of a microcontroller, which controls the sweep of the sweep generator and receives said amplitude and phase output signals from the amplitude and phase detector.

5. The device according to claim 4, wherein said microcontroller is at the same time connected to an LCD display and/or a wireless transmission module.
